Europäisches Patentamt

European Patent Office

Office européen des brevets

(11) Veröffentlichungsnummer: **0 015 550**
**A1**

(19)

(12) # EUROPÄISCHE PATENTANMELDUNG

(21) Anmeldenummer: 80101121.4

(22) Anmeldetag: 05.03.80

(51) Int. Cl.³: **G 01 N 11/16**, G 01 N 33/48

(30) Priorität: 05.03.79 DE 2908469

(43) Veröffentlichungstag der Anmeldung: 17.09.80
Patentblatt 80/19

(84) Benannte Vertragsstaaten: **AT BE CH FR GB IT LU NL SE**

(71) Anmelder: **Dr. Eduard Fresenius Chemisch-pharmazeutische Industrie KG, Gluckensteinweg 5, D-6380 Bad Homburg v.d.H. 1 (DE)**

(72) Erfinder: **Husar, Dieter, Dr. rer. nat. Dipl.-Phys., Holzhäuser Strasse 6, D-6380 Bad Homburg v. d. Höhe (DE)**

(74) Vertreter: **KUHNEN & WACKER Patentanwaltsbüro, Schneggstrasse 3-5 Postfach 1729, D-8050 Freising (DE)**

(54) Verfahren und Vorrichtung zur Bestimmung der visko-elastischen Eigenschaften von Fluiden.

(57) Die Erfindung betrifft ein Verfahren und eine Vorrichtung zur Bestimmung der visko-elastischen Eigenschaften insbesondere von Blut oder Blutplasma. Die Blutprobe (3a) wird in einem Probengefäss (1) durch einen schwingenden Körper (2) beaufschlagt. Bei einsetzender Blutgerinnung ändert sich das Schwingungsverhalten des durch den schwingenden Körper (2) und seine elastische Lagerung (5) gebildeten Schwingsystems, welches sich gewissermassen über die Blutprobe (3a) gegen die festen Wände des Probengefässes (1) abstützt. Dabei wird entweder die Anregungsfrequenz des Schwingsystems mit dem Körper (2) so nachgeführt, dass die Anregung ständig in der sich verschiebenden Resonanz des Schwingsystems bleibt, oder es wird die Anregungsfrequenz über die Resonanzfrequenz hinweg verändert. Hierzu wird die Anregungsfrequenz in kurzen Abständen über einen Frequenzbereich gefahren, der die Resonanzfrequenz enthält, um so in zeitlicher Folge Frequenzlage und Amplitudenhöhe in der Resonanz abzutasten. Während die Verschiebung der Frequenzlage der Resonanzfrequenz ein Mass für die Änderung der Elastizität der Blutprobe ist, ist die Änderung der Amplitude in der Resonanz ein Mass für die Änderung der Viskosität der Blutprobe. Auf diese Weise lassen sich die viskosen und elastischen Änderungen der Blutprobe feinfühlig und frei von Störungen erfassen und so sehr detaillierte Rückschlüsse auf die Konsistenz des Blutes ziehen.

EP 0 015 550 A1

# KUHNEN & WACKER

## PATENTANWALTSBÜRO ——— 0015550

REGISTERED REPRESENTATIVES BEFORE THE EUROPEAN PATENT OFFICE

Dr. Eduard Fresenius

Chemisch-pharmazeutische

Industrie KG

6380 Bad Homburg

PATENTANWÄLTE

R.-A. KUHNEN*, DIPL.-ING.
W. LUDERSCHMIDT**, DR. DIPL.-CHEM
P.-A. WACKER*, DIPL.-ING., DIPL.-WIRTSCH.-ING.

55 FR 0330 2/st

Verfahren und Vorrichtung zur Bestimmung
der visko-elastischen Eigenschaften von
Fluiden

Die Erfindung betrifft ein Verfahren zur Bestimmung der visko-elastischen Eigenschaften von Fluiden und/oder polymerisierenden Gemischen, insbesondere von gerinnenden Flüssigkeiten wie Blut oder Blutplasma, und deren zeitlichen Änderungen, nach dem Oberbegriff des Anspruchs 1, sowie zur Durchführung des Verfahrens besonders geeignete Vorrichtungen.

Insbesondere zur Bestimmung des Gerinnungsverhaltens von Blut oder Blutplasma ist eine Reihe von sog. Globalbestimmungsmethoden bekannt, die den Gerinnungsablauf zeitlich verfolgen und somit Aufschlüsse über Ablauf und Umfang der Gerinnung und damit Rückschlüsse auf Eigenschaften des Blutes oder Blutplasmas ermöglichen.

Aus einem Aufsatz "Einfache, tragbare, kurvenschreiben-de Gerinnungsmaschine für Notfallstation, Operations-saal und Krankenzimmer" von K. N. v. Kaulla und E. v. Kaulla in der Zeitschrift "Verh. Dt. Ges. inn. Med.",

BÜRO 6370 OBERURSEL**
LINDENSTRASSE 10
TEL 06171 56849
TELEX 4186343 real d

BÜRO 8050 FREISING*
SCHNEGGSTRASSE 3-5
TEL 08161 62091
TELEX 526547 pawa d

ZWEIGBÜRO 8390 PASSAU
LUDWIGSTRASSE 2
TEL 0851 36616

—— TELEGRAMMADRESSE PAWAMUC   POSTSCHECK MÜNCHEN 1360 52 802 ——

1977, S. 1133 bis 1188 ist beispielsweise ein Verfahren bekannt, bei dem die Änderung der Konsistenz von Blut oder Blutplasma bei der Ausbildung des Fibrinnetzes erfaßt wird. Hierzu taucht in ein Probengefäß ein Stempel ein, der bei einer oszillierenden Auf- und Abbewegung das Fluid der Blut- oder Plasmaprobe verdrängt und somit einem Fließvorgang unterwirft. Die oszillierende Bewegung des Stempels ist dabei außerordentlich klein, um die Bildung dünner Fibrinfäden nicht durch einen makroskopisch ausgeprägten, erzwungenen Strömungsvorgang mit starker Druckbeaufschlagung zu gefährden. Der oszillierende Antrieb des Stempels erfolgt durch Befestigung an der Membran eines Lautsprechers, an dem ein Brummton mit einer konstanten Frequenz von 90 Hz erzeugt wird. Mit zunehmender Viskosität der Fluidprobe steigt der Widerstand des Fluids gegen die oszillierende Bewegung des Stempels, so daß die Schwingungsamplitude der Lautsprechermembran abfällt; diese Erhöhung des Widerstands auf die erzwungene Schwingung des Stempels wird durch eine Änderung der Durchgangsintensität des Stromes durch den Lautsprecher erfaßt.

Der hauptsächliche Nachteil dieses Verfahrens besteht darin, daß in das Meßergebnis lediglich die Änderung des dem Stempel durch die Fluidprobe entgegengesetzten Widerstandes gegen seine Oszillationsbewegung eingeht, letztlich also eine von der Änderung der Viskosität und Elastizität gemeinsam abhängige, untrennbar mit diesen beiden Parametern verknüpfte Größe, die somit keinen Aufschluß über Änderungen der Viskosität oder der Elastizität alleine geben kann. Nachteilig ist weiterhin, daß diese Größe über die Änderung der Amplitude der Mikroschwingung erfaßt wird, was eine äußerst empfindliche Messung voraussetzt, um insbesondere den spontanen Beginn der Konsistenzänderung zu erfassen, da die Amplitude ohnehin minimal ist.

0015550

In der älteren deutschen Patentanmeldung P 27 41 060.2-52 ist ein Verfahren vorgeschlagen worden, mit dem die zeitliche Änderung der Konsistenz einer Blutprobe im Prinzip dadurch bestimmt wird, daß die Probe Teil eines mit konstanter Frequenz angeregten Schwingsystems bildet, dessen Schwingungsamplitude durch Änderung der Konsistenz der Probe verändert wird, und diese Änderung erfaßt wird. Hierzu ist ein Probengefäß am oberen Ende eines elastischen Stabes befestigt, dem durch Anregung an seinem unteren Ende eine Orbitalschwingung aufgezwungen wird, während in das Probengefäß und in die Blutprobe ein ortsfester Stempel eintaucht. Zunächst wird die Resonanzfrequenz des vom elastischen Stab, dem Probengefäß und der flüssigen Blutprobe gebildeten Schwingsystems ermittelt, und sodann eine Anregungsfrequenz gewählt, welche knapp neben dieser Resonanz- oder Eigenfrequenz auf derjenigen Seite liegt, zu der sich die Resonanzfrequenz des Schwingsystems bei Ausbildung eines elastischen Fibrinnetzes in der Blutprobe verschiebt. Dadurch erfolgt die Anregung des Schwingsystems zunächst außerhalb der Resonanzfrequenz, so lange keine Fibrinbildung vorliegt. Sobald die Fibrinbildung einsetzt, ändert sich durch die zunehmende Elastizität des Fibrinnetzes die Federkonstante des Schwingsystems und verschiebt dessen Resonanzfrequenz zur Anregungsfrequenz der erzwungenen Schwingung hin, so daß in der Schwingung eine deutliche Resonanzspitze auftritt. Bei weiterer Verfestigung des Fibrinnetzes und damit weiterer Verschiebung der Resonanzfrequenz wandert diese über die Anregungsfrequenz der erzwungenen Schwingung hinaus, so daß die abgegriffene Amplitude des Schwingsystems bei der Verschiebung der Resonanzfrequenz in Richtung auf die Anregungsfrequenz zunächst markant ansteigt und dann wieder stark abfällt.

Dieses Verfahren erzielt zwar eine von Störungen weitgehend unabhängige Verfeinerung gegenüber einem aus der DE-OS 20 19 341 bekannten Verfahren, bei dem das sich

0015550

ausbildende Fibrinnetz einen in die Blutprobe eintauchenden Meßkörper zunehmend drehend mitnimmt: Die Messung im Resonanzbereich ergibt eine scharfe, deutliche Amplitudenspitze, die eindeutig erkennbar ist und in der Regel von Störungen nicht überlagert werden kann. Jedoch leidet die Meßgenauigkeit bei diesem Verfahren darunter, daß das Auftreten der Resonanzspitze nicht nur von dem tatsächlichen Beginn der Fibrinbildung abhängt, sondern auch von dem Anfangsabstand zwischen der Resonanzfrequenz des Schwingsystems und der Anregungsfrequenz; je größer dieser Abstand ist, umso weiter muß die Fibrinbildung bereits fortgeschritten sein, bevor überhaupt die Resonanz auftritt. Der Abstand zwischen der Resonanzfrequenz des Schwingsystems zu Beginn der Messung und der Anregungsfrequenz ist jedoch von einer Vielzahl von Einflüssen abhängig, die gerade bei dem anzustrebenden geringen Abstand deutlich fühlbare Änderungen dieses Frequenzabstandes bewirken können.

Zudem wird - je nach dem Anfangsabstand zur Resonanzfrequenz des Schwingsystems mehr oder weniger genau - insbesondere der Beginn der Fibrinbildung durch die Resonanzspitze erfaßt, während nach Überlaufen der Anregungsfrequenz durch die Resonanzfrequenz des Schwingsystems in jedem Falle ein starker Amplitudenabfall auftritt, der konkrete Rückschlüsse auf den weiteren Verlauf der Fibrinbildung nur schwer zuläßt. Gerade aber durch Ausnutzung der Resonanz soll dieses Verfahren ein eindeutig erfaßbares Kriterium für die Fibrinbildung schaffen und ist somit auch vorrichtungstechnisch nicht auf eine Erfassung ganz minimaler Änderungen außerhalb der Resonanz eingerichtet.

Ein ganz wesentlicher Nachteil auch dieses bekannten Verfahrens besteht weiterhin darin, daß die elastischen Zustandsänderungen und die Änderungen der Viskosität der Fluidprobe aufgrund des Meßprinzips untrennbar miteinander verknüpft sind. Eine konkrete Aussage über die Änderung der Viskosität einerseits und der Elastizität andererseits läßt sich daher nicht machen. Im Rahmen der vorliegenden Erfindung durchgeführte Versuche haben jedoch gezeigt, daß sich gerade durch eine getrennte Erfassung der Viskositätsänderungen einerseits und der Elastizitätsänderungen andererseits Aussagen über die wichtigen Gerinnungsparameter F I (Fibrinogen), F XIII (fibrinstabilisierender Faktor) und Thrombozytenzahl auf besonders klare Weise quantifizieren lassen.

Somit liegt der Erfindung die Aufgabe zugrunde, ausgehend von dem älteren Vorschlag gemäß der deutschen Patentanmeldung P 27 41 060.2-52 ein Verfahren zu schaffen, welches ebenfalls zur Ausschaltung von Störeinflüssen auf die Messung Resonanzerscheinungen eines die Fluidprobe umfassenden Schwingsystems nutzt, dabei aber gleichzeitig eine exakte getrennte Erfassung sowohl der Änderung der Elastizität als auch der Änderung der Viskosität, und zwar beides sowohl hinsichtlich des genauen Beginns als auch hinsichtlich des weiteren Verlaufs, ermöglicht.

Die Lösung dieser Aufgabe erfolgt durch die kennzeichnenden Merkmale des Anspruchs 1.

Dadurch, daß die Amplitude in der jeweiligen Resonanzfrequenz erfaßt wird, also sozusagen stets im Resonanzbereich des Schwingsystems gemessen wird, stehen stets klare und gut erkennbare, durch Überlagerungsstörungen nicht merklich beeinflußte Meßgrößen zur Verfügung. Die Frequenzlage der Resonanzfrequenz gibt dabei den Verlauf der Verstimmung der Resonanzfrequenz des Schwingsystems und damit der Änderung der Elastizität der Fluidprobe einwandfrei wieder. Andererseits ergibt die Amplitude in der Resonanzfrequenz ein Maß für die fortschreitende Dämpfung der Schwingung und damit für die Änderung der Viskosität der Fluidprobe.

Es stehen hierzu zwei grundsätzliche Möglichkeiten zur Verfügung: Entweder wird periodisch in ausreichend kurzen Zeitabständen der Bereich der möglichen Lage der Resonanzfrequenz mit der Anregungsfrequenz überfahren ("Wobbelverfahren"), oder es wird die Anregungsfrequenz dauernd der Resonanzfrequenz des Schwingsystems nachgestellt und so stets in der Resonanzfrequenz gemessen. Im ersten Falle ergibt sich bei entsprechender Kurvenaufzeichnung pro Abtastlauf der Anregungsfrequenz eine die Resonanzspitze enthaltende Amplitudenkurve, wobei jedoch erfindungsgemäß nicht die Anregungsfrequenz konstant gehalten und die Verstimmung der Resonanzfrequenz abgewartet wird, sondern in entsprechend kurzen zeitlichen Abständen durch den Abtastlauf der veränderlichen Anregungsfrequenz eine Kurvenschar erhalten wird, die mit Beginn der Fibrinbildung einen Abfall der Amplitudenhöhe der Resonanzspitzen von Kurve zu Kurve erkennen läßt, was der Erhöhung der Viskosität der Fluidprobe entspricht, sowie weiterhin eine Verschiebung der Frequenzlage der Resonanzspitze erkennen läßt, was der Verschiebung der Resonanzfrequenz und damit der Änderung der Elastizität der Fluidprobe entspricht. Im anderen Fall kann einerseits etwa durch einen Schrieb festgehalten werden, in welcher Weise die Anregungsfrequenz über der Zeit verändert worden ist, um ständig in der Resonanzfrequenz zu arbeiten, was der Verschiebung der Resonanzfrequenz und damit der Änderung der Elastizität der Fluidprobe entspricht, während gleichzeitig und gegebenenfalls kontinuierlich die dabei anfallende Amplitude abgegriffen werden kann, deren allmählicher Abfall der zunehmenden Dämpfung des Schwingsystems und damit der Änderung der Viskosität der Fluidprobe entspricht.

Beide Möglichkeiten ergeben eine fast verzögerungsfreie Erfassung des Beginns von Änderungen sowohl der Elastizität als auch der Viskosität der Fluidprobe, zumal die Abtastfrequenz mit der Anregungsfrequenz bei der erstgenannten Möglichkeit je nach Bedarf klein genug gehalten werden kann, um Zeitverzögerungen bei der Erfassung von Änderungen der Resonanzamplitudenlage und -amplitudenhöhe zu Beginn der Fibrinbildung auf einem in der Praxis vernachlässigbaren Maß zu halten. In beiden Fällen ergibt sich eine bei Bedarf kontinuierliche oder quasi-kontinuierliche Erfassung des weiteren Verlaufs der Elastizitätsänderung und der Viskositätsänderung, was weitere Rückschlüsse auf Eigenschaften des untersuchten Blutes zuläßt.

Vorrichtungstechnisch wird die gestellte Aufgabe durch die kennzeichnenden Merkmale der Ansprüche 11 und 13 gelöst, während die Ansprüche 12 und 14 bis 21 vorteilhafte Weiterbildungen der Vorrichtung zum Inhalt haben.

Weitere Einzelheiten, Merkmale und Vorteile der Erfindung ergeben sich aus der nachfolgenden Beschreibung von Ausführungsbeispielen anhand der Zeichnung. Es zeigt

Fig. 1    eine schematisch vereinfachte Vorrichtung zur Durchführung einer ersten Ausführungsform des erfindungsgemäßen Verfahrens,

Fig. 2    in einer Darstellung entsprechend Fig. 1 eine zur Durchführung einer zweiten Ausführungsform des erfindungsgemäßen Verfahrens geeignete Vorrichtung,

Fig. 3    eine bei der ersten Ausführungsform des erfindungsgemäßen Verfahrens beispielsweise erhaltene Kurvenschar zur Veranschaulichung der Meßergebnisse,

Fig. 4 und 5    Meßkurven von Normalblut für die Änderung der Resonanzfrequenz bzw. der Amplitude, wie sie mit der zweiten Ausführungsform der Erfindung unmittelbar und mit der ersten Ausführungsform der Erfindung mittelbar erhalten werden können und

Fig. 6 einen beispielhaften Verlauf der Abtastung bei der ersten Ausführungsform der Erfindung.

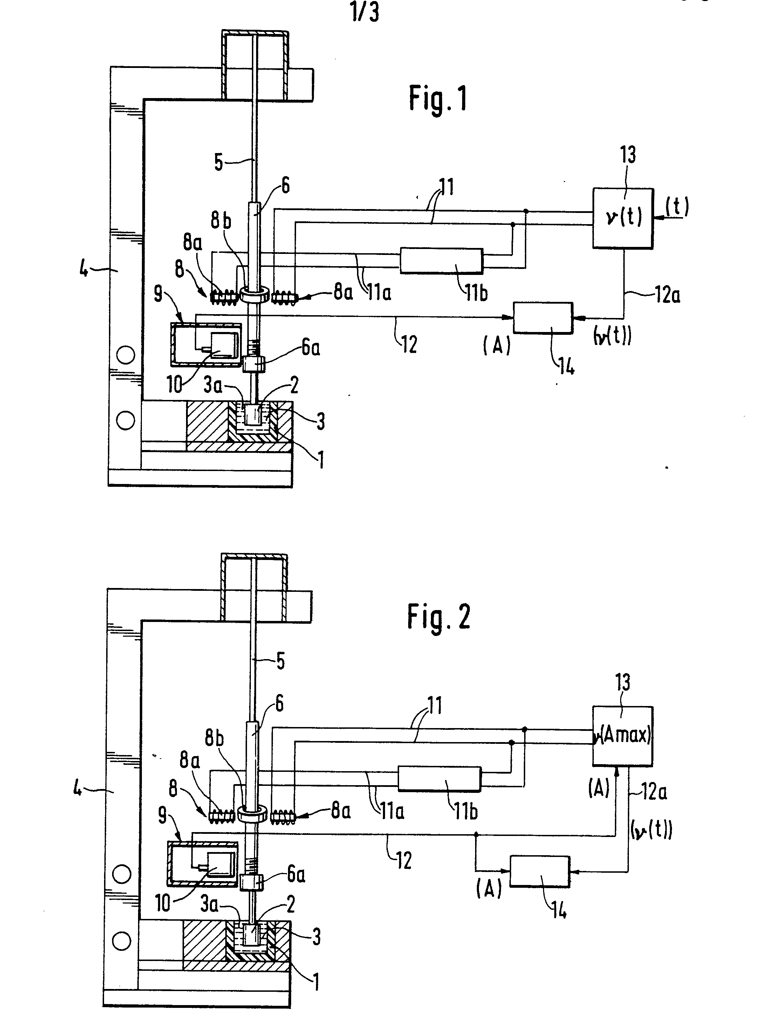

In den Fig. 1 und 2 ist ein möglicher grundsätzlicher Aufbau einer Vorrichtung zur Durchführung des erfindungsgemäßen Verfahrens veranschaulicht, wobei gleiche Teile mit gleichen Bezugszeichen versehen sind. Dabei ist mit 1 ein Probengefäß bezeichnet, welches im Beispielsfalle hohlzylindrisch ausgebildet ist und in welches ein im Beispielsfalle zylindrischer Körper 2 derart eingreift, daß zwischen der Innenwand des Probengefäßes 1 und der Außenwand des Körpers 2 ein Zwischenraum 3 verbleibt, in dem sich eine Fluidprobe 3a, beispielsweise eine geringe Blutmenge, befindet. Dabei taucht der Körper 2 vollständig in der Fluidprobe 3a unter. Die Größe des Zwischenraumes 3 zwischen dem Körper und der Innenwand des Probengefäßes 1 ist nicht kritisch, so lange natürlich keine Behinderung der relativen Schwingbewegung zwischen Probengefäß 1 und Körper 2 auftritt, und kann so groß gewählt werden, als dadurch praktikable Abmessungen des Probengefäßes nicht überschritten werden. Das Probengefäß 1 und der Körper 2 können leicht auswechselbar gehalten sein und bei Herstellung aus einem billigen Werkstoff wie einem geeigneten Kunststoff nach jeder Messung weggeworfen werden, oder aber aus einem dauerhaften Werkstoff wie etwa V2A-Stahl bestehen und nach jeder Messung gereinigt werden. Das Probengefäß 1 kann weiterhin mit weiteren Probengefäßen an einem Gleithalter gelagert sein, der nach jeder Messung im Takt derart gegenüber dem Körper 2 verschoben wird, daß der Körper 2 - gegebenenfalls nach seiner Auswechselung - in ein neues Probengefäß 1 eintaucht. Wie bei Mehrfachküvetten bekannt ist, kann eine solche Mehrfachanordnung als Drehteller, Schiebeschiene od. dgl. ausgebildet werden. Neben einem Rationalisierungseffekt bezüglich des Meßvorgangs ergibt eine solche Mehrfachanordnung auch eine einfache Möglichkeit zur Anbringung einer Temperaturregeleinrichtung, welche die Temperatur in den Probengefäßen 1 stets auf Körpertemperatur hält. Selbstverständlich ist eine solche Temperaturregeleinrichtung auch bei einzelnen Probengefäßen 1 verwendbar. Da solche Temperaturregeleinrichtungen an sich bekannt sind, erübrigt sich ein weiteres Eingehen hierauf.

Im Beispielsfalle ist das Probengefäß 1 fest an dem mit 4 bezeichneten Gerätegestell angeordnet, welches in geeigneter Weise klappbar sein kann, während der Körper 2 an einem elastischen Stab 5 aufgehängt ist. Grundsätzlich könnte auch das Probengefäß 1 am Stab 5, etwa an dessen oberem Ende, befestigt werden, während der Körper 2 gestellfest gehalten wird, jedoch bereitet hier die Verwendung auswechselbarer Probengefäße 1 und Körper 2 größere Schwierigkeiten. In jedem Falle ist der Körper 2 gegenüber dem Probengefäß 1 infolge der biegeelastischen Ausbildung der Abstützung entweder des Körpers 2 oder des Probengefäßes 1 - im Beispielsfalle des Stabes 5 - beweglich, wobei im Falle einer Schwingungsanregung eine Relativschwingung des nicht gestellfesten Teiles - Probengefäß 1 oder Körper 2 - unter Beaufschlagung der Fluidprobe 3a erfolgen kann. Zur Einstellung einer geeigneten Resonanzfrequenz ist der Stab 5 in der Nachbarschaft seines freien Endes mit einer Verdickung 6 oder einer entsprechenden Manschette sowie einem beispielsweise durch ein Schraubgewinde höheneinstellbaren Justiergewicht 6a versehen.

Bevorzugt an der dem freien Ende des Stabes 5 benachbarten Hälfte seiner Längenerstreckung ist ein Schwingantrieb 8 vorgesehen, der im Beispielsfalle zur Erzeugung einer Orbitalschwingung aus vier senkrecht zueinander angeordneten Elektromagneten 8a besteht, die bei Erregung in geeigneter Weise auf das eisenhaltige Material des Stabes 5 oder im Beispielsfalle auf einen etwa durch Klebung am Stab 5 befestigten Ringmagneten 8b einwirken und diesen anziehen oder abstoßen. In der Zeichnung sind zur Vereinfachung der Darstellung nur zwei einander gegenüberliegende Elektromagnete 8a dargestellt, wie dies zur Erzeugung einer translatorischen Schwingung in einer Ebene erforderlich ist; eine Orbitalschwingung kann in besonders einfacher Weise allenfalls durch nur zwei Elektromagnete 8a erzeugt werden, die dann aber in einem Winkel von 90° zueinander liegen.

Möglichst nahe am freien Ende des Stabes 5, wo die Schwingungsausschläge am größten sind, ist ein Amplitudenfühler oder Schwingungssensor 10 beispielsweise in Form eines Hallgenerators od. dgl. angeordnet, der auf die Annäherung des benachbarten Abschnittes des Stabes 5 anspricht. Der Schwingungssensor 10 ist im Beispielsfalle vergleichsweise eng benachbart zu den bevorzugt ebenfalls in der Nähe des freien Endes des Stabes 5 angeordneten Elektromagneten 8a montiert und von einer gehäuseartigen Magnetfeldabschirmung 9 umgeben, welche zum Schutz vor Einwirkungen von den Elektromagneten 8a her dient. Bei Schwingung des Stabes 5 mit einer Komponente in Richtung auf den Schwingungssensor 10 erfaßt dieser die Annäherung und Entfernung des Stabes 5 und gibt entsprechend dem momentanen Abstand des Stabes 5 abstandsproportionale Signale über eine Meßleitung 12 ab.

Die konstruktiven Einzelheiten der dargestellten Vorrichtung dienen lediglich zur Veranschaulichung einer möglichen, bevorzugten Ausführungsform, von der vielfache Abweichungen möglich sind. So kann beispielsweise die Abnahme der Schwingungsamplitude statt über einen Hallgenerator als Schwingungssensor 10 auch auf jede andere geeignete Weise erfolgen, wobei aber in jedem Falle eine berührungsfreie Abnahme der Amplitude bevorzugt ist, um die freie Schwingung des Körpers 2 durch Rückwirkungen der Meßwertabnahme nicht oder jedenfalls nicht wesentlich zu beeinträchtigen.

Anstelle des elektromagnetischen Schwingantriebes 8 kann auch ein entsprechender mechanischer Antrieb verwendet werden, wie er beispielsweise aus der DE-OS 20 19 341 ersichtlich ist, auf die wegen weiterer Einzelheiten insoweit ausdrücklich Bezug genommen wird. Weiterhin ist nicht zwingend erforderlich, dem Stab 5 eine Orbitalbewegung aufzuzwingen, sondern kann auch eine lineare Oszillationsbewegung als Schwingung verwendet werden. Entsprechend brauchen das Probengefäß 1 und der Körper 2 nicht hohlzylindrisch oder zylindrisch ausgebildet sein, und kann dementsprechend der Zwischenraum 3 auch abweichend von der Ringform etwa eben ausgebildet sein und quer zur Schwingungsrichtung der Oszillation liegen oder mit dieser jedenfalls einen endlichen Winkel einschließen. Auch braucht nicht zwangsläufig die Innenwand des Probengefäßes 1 der Form der Außenwand des Körpers 2 angepaßt zu sein, so lange Abweichungen durch einen ausreichend breiten Zwischenraum 3 kompensiert werden. Im Falle einer Orbitalbewegung ist der Stab 5 oder ein die Elastizität ergebender Stababschnitt bevorzugt mit rundem Querschnitt ausgebildet, weist also jedenfalls in beiden Achsen der Orbitalbewegung gleiche Elastizität auf; im Falle einer linearen Oszillationsbewegung kann der Stab 5 auch als flaches Federblatt ausgebildet sein oder in einem Abschnitt ein solches enthalten bzw. an einem solchen angeschlossen sein. Die Ausbildung der Lagerung des Stabes 5 ist ebenfalls weitgehend freigestellt und hängt von dessen Ausbildung im einzelnen sowie von der Art der erzwungenen Schwingung ab.

Im Falle eines elektromagnetischen Schwingantriebes 8 erfolgt die Strombeaufschlagung der Elektromagnete 8a über Leitungen 11 von einem Steuerteil 13 aus. Das

Steuerteil 13 steuert den Stromdurchfluß durch die Spulen der Elektromagnete 8a, wobei eine unmittelbare Steuerung über die Leitungen 11 nur an einem Elektromagneten 8a erfolgt, während die übrigen Elektromagnete 8a, wie in der Zeichnung für einen Elektromagneten dargestellt ist, über Zweigleitungen 11a und je einen Phasenschieber 11b derart angesteuert werden, daß unabhängig von der Geschwindigkeit der Ansteuerung über die Hauptleitungen 11 stets eine entsprechend phasenverschobene Ansteuerung der weiteren Elektromagnete 8a derart erfolgt, daß eine saubere Schwingungsanregung gewährleistet ist.

Wesentlich zur Durchführung des erfindungsgemäßen Verfahrens ist, daß die Fluidprobe 3a von der Wand eines schwingenden Körpers beaufschlagt wird, der Teil eines Schwingsystems mit definierter Resonanzfrequenz ist, die durch Rückwirkungen vom Fluid her von den Elastizitätseigenschaften des Fluids abhängt. Im Prinzip kann dabei, wie beim Ausführungsbeispiel, der elastisch gelagerte Körper 2 angetrieben werden, oder aber auch das starr gelagerte Probengefäß, so daß es über die Fluidprobe 3a den elastisch gelagerten Körper 2 mitnimmt und so zu entsprechenden Schwingungen anregt. In jedem Falle wirkt das visko-elastische Fluid 3a im Bereich eines Schwingungsbauches einer angeregten Schwingung entsprechend seinen Elastizitätseigenschaften als Federabstützung und entsprechend seinen Viskositätseigenschaften als Dämpfungsglied auf das Schwingsystem ein. Bei Änderungen der Elastizitätseigenschaften des visko-elastischen Fluids 3a ergibt sich somit eine Änderung der Eigenfrequenz des Schwingsystems, also eine Änderung seiner Gesamt-Federkonstante, während sich bei Änderungen der Viskosität des Fluids 3a eine Änderung der Dämpfung des Schwingsystems ergibt.

Die Orbitalpendelbewegung des sich bewegenden Stabes 5 erfolgt derart, daß die dadurch erzwungene Amplitude in der Resonanz in einem Bereich von 1 bis 100 $\mu$m, vorzugsweise von 10 bis 50 $\mu$m liegt. Wird die Amplitude größer, so besteht die Gefahr, daß das Fibrinnetz nicht mehr elastisch mitschwingen kann und abreißt oder in sich zerreißt. Andererseits sind die Amplituden in der Resonanz unterhalb 1 $\mu$m zu klein, um die erzeugten Effekte mit einem üblicherweise vertretbaren Meßaufwand messen zu können.

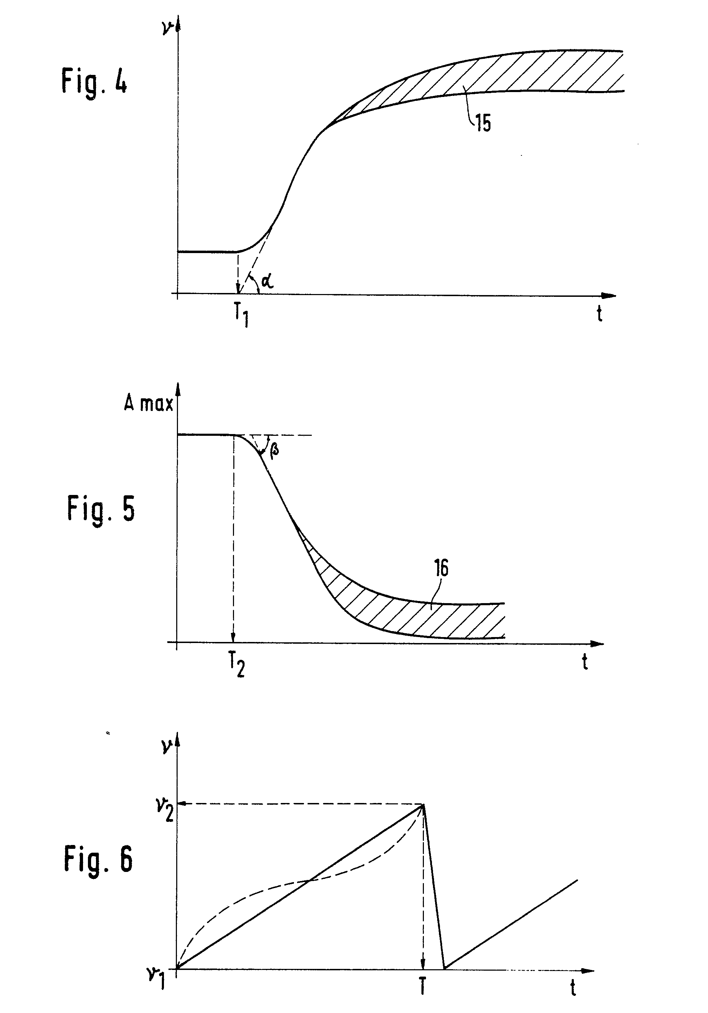

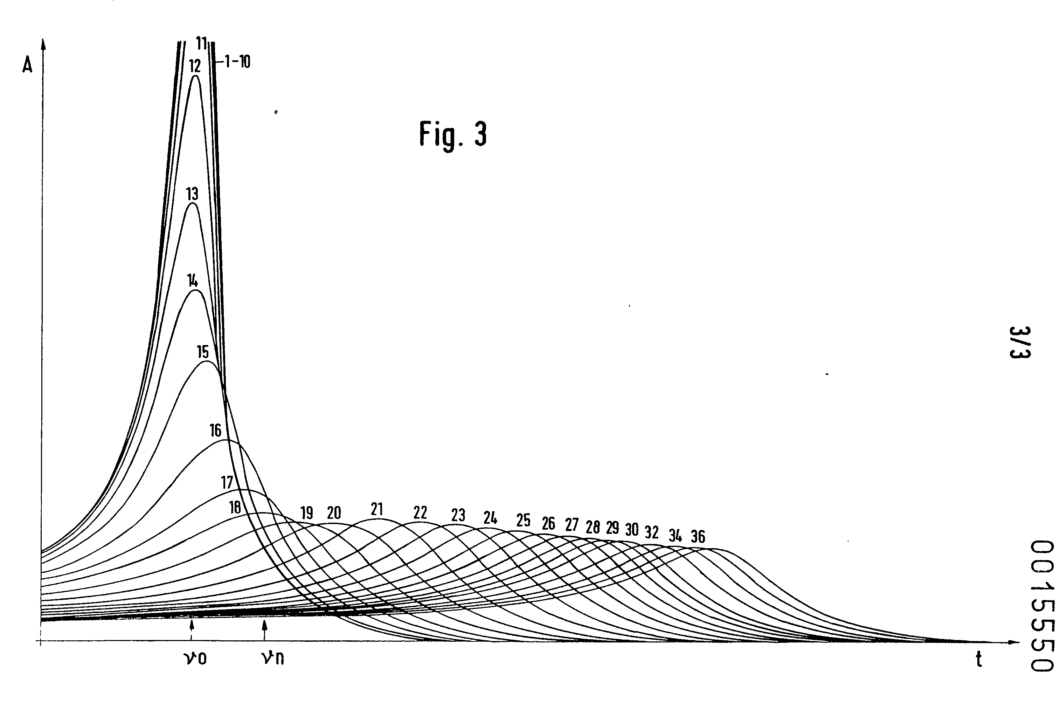

Im Falle der Anordnung gemäß Fig. 1 stellt das Steuerteil 13 die Anregungsfrequenz des Schwingantriebes 8 in Abhängigkeit von der Zeit t in vorbestimmter Weise ein. Hierzu kann im Steuerteil 13 beispielsweise eine Kippstufe vorgesehen sein, die einen sägezahnartigen Verlauf der Anregungsfrequenz zwischen einer unteren Frequenz $y_1$ und einer oberen Frequenz $y_2$ in der aus Fig. 6 ersichtlichen Weise ergibt; der Frequenzverlauf braucht dabei nicht, wie in Fig. 6 mit ausgezogener Linie veranschaulicht ist, linear zu sein, sondern es ist auch ein abweichender Verlauf beispielsweise gemäß der gestrichelten Linie in Fig. 6 möglich, so daß etwa ein amplituden-antiproportional gesteuerter Frequenzverlauf eingestellt werden kann, so daß die Abtastung in der Resonanz besonders langsam veränderlich ist, was bei nicht-linearem Schwingungsverhalten der Fluidprobe von Vorteil sein kann. In der Zeitspanne C, die zum Durchlaufen des Abtastfrequenzbereiches zwischen $y_1$ und $y_2$ benötigt wird, und die typischerweise im Falle von Blut als Fluid 3a zwischen etwa 0,5 und 200 Sekunden liegen kann, erfolgt somit ein Überfahren eines vorbestimmten Frequenzbereiches, wonach die Frequenz schnell auf die Anfangsfrequenz des Abtastbereiches zurückgeführt und erneut mit der Abtastung begonnen wird. Selbstverständlich kann die Abtastung dabei von der unteren Frequenz zur oberen Frequenz des Abtastbereiches oder umgekehrt geführt werden, und kann anstelle einer schnellen Rückstellung auf die Anfangsfrequenz auch ein Überfahren des Abtastbereiches in der Gegenrichtung unter Durchführung einer Nutzabtastung erfolgen.

Der Frequenzbereich der Abtastung zwischen $\gamma_1$ und $\gamma_2$ ist in jedem Falle so gewählt, daß mit Sicherheit die jeweilige Resonanzfrequenz des durch den Stab 5 und den Körper 2 gebildeten Schwingsystemes überfahren wird. Hierzu kann der Frequenzbereich der Abtastung zweckmäßig zwischen etwa dem 0,1-fachen und dem 3-fachen der Anfangsresonanzfrequenz des Schwingsystems liegen, zur Vermeidung überflüssiger Totzeiten wird jedoch vorteilhaft ein Frequenzbereich der Abtastung zwischen etwa dem 0,8-fachen und dem 1,8-fachen der Anfangsresonanzfrequenz gewählt, wobei zu berücksichtigen ist, daß im Falle einer Gerinnung des durch Blut gebildeten Fluids 3a durch Ausbildung des Fibrinnetzes die Federkonstante des Schwingsystems härter wird und damit die Resonanzfrequenz ansteigt. Bei einer Anfangsresonanzfrequenz des Schwingsystems von beispielsweise 80 Hz kann somit der Abtastfrequenzbereich oder "Wobbel"-Bereich zwischen $\gamma_1$ = 65 Hz und $\gamma_2$ = 140 Hz gewählt werden.

Bei einer derartigen Abtastung ermittelte Amplitudenkurven, wie sie am Schwingungssensor 10 erfaßt werden, sind in Fig. 3 veranschaulicht. Eine für die Auswertung günstige Darstellung dieser Amplitudenkurven läßt sich gemäß Fig. 3 dadurch erzielen, daß an der Leitung 12 und einer Leitung 12a vom Steuerteil 13 her zur Zuordnung der jeweiligen Anregungsfrequenz ein X-Y-Schreiber 14 angeschlossen wird, dessen Abszissenstift mit der Zeit und dessen Ordinatenstift mit der momentanen Amplitude läuft, wobei zu Beginn jeder Abtastung in einer Abtastrichtung der Abszissenschreiber wieder auf Null zurückgefahren wird, um die aufeinanderfolgenden Abtastkurven über einem abtastungsspezifischen Zeitmaßstab aufzutragen. Da die Zuordnung der Anregungsfrequenz im Verlauf der Abtastung zur Zeit im Steuerteil 13 vorbestimmt ist, beispielsweise gemäß der ausgezogenen Linie in Fig. 6 linear ist, kann somit jedem Zeitpunkt auf der Abszisse des Schreibers eine bestimmte Anregungsfrequenz zugeordnet werden, nämlich diejenige Anregungsfrequenz, die das Steuerteil 13 nach einer vorbestimmten Zeitspanne ab Beginn der Abtastung erreicht hat. Somit ist die Abszisse zugleich ein Maßstab für die jeweilige Anregungsfrequenz.

Wie aus Fig. 3 ersichtlich ist, überdecken sich im Zuge der Messung die ersten Abtastkurven, im Beispielsfalle die Kurven der 1. bis zur 10. Abtastung. Dies läßt erkennen, daß während der entsprechenden Abtastzeit kein Parameter des Schwingsystems einer Änderung unterworfen wurde, so daß sich stets bei der gleichen Anregungsfrequenz entsprechend der Anfangseigenfrequenz des Schwingsystems die Resonanz mit im wesentlichen gleicher Amplitude ergibt. Zwischen der 10. Abtastkurve und der 11. Abtastkurve ist eine erste Veränderung festzustellen, da die Resonanzamplitude nicht mehr die ursprüngliche Höhe erreicht, was einen Rückschluß auf eine wirksam werdende erhöhte Dämpfung zuläßt; die nachfolgenden Kurven zeigen eine zunächst steil und dann flach abfallende Amplitudenhöhe, was dem Verlauf der Dämpfung durch Erhöhung der Viskosität des Probefluids entspricht. Gleichzeitig ist festzustellen, daß die Lage des Amplitudenmaximums sich gemäß Fig. 3 zunehmend nach rechts verschiebt, also im Abtastzyklus immer später auftritt, was einem Auftreten bei entsprechend höherer Frequenz zugeordnet werden kann. In Fig. 3 ist mit $\gamma_0$ die Lage der Anfangsresonanzfrequenz des Schwingsystems und mit $\gamma_n$ im Beispielsfalle die Lage der Eigenfrequenz des Schwingsystems zum Zeitpunkt der Aufnahme der 18. Abtast- oder Meßkurve, also bei der 18. Abtastung, veranschaulicht, wodurch sich deutlich die Verschiebung der Resonanzfrequenz ergibt. Diese Verschiebung der Resonanzfrequenz hat ihre Ursache in einer Änderung der Elastizität des Fluids 3a und damit der Gesamtfederkonstante des Schwingsystems.

Auf diese Weise lassen sich somit Änderungen sowohl der viskosen als auch der elastischen Eigenschaften des visko-elastischen Fluids 3a getrennt hinsichtlich Beginn der Änderung und Verlauf der Änderung bestimmen.

Selbstverständlich liegt gemäß der Steuerung des Steuerteiles 13 in Abhängigkeit von der Zeit t auch der Zeitpunkt vom Beginn der Abtastung gerechnet an fest, an dem jede einzelne Abtastkurve aufgenommen wurde, so daß sich aus der in Fig. 3 enthaltenen Information der Verlauf der Änderung der Resonanzfrequenz des Schwingsystems über der Zeit gemäß Fig. 4 und der Verlauf der Änderung der maximalen Amplitudenhöhe bei der Resonanz gemäß Fig. 5 ermitteln läßt. Anstelle einer Ermittlung der Kurven gemäß Fig. 4 und 5 aus aus Fig. 3 herauslesbaren Meßpunkten ist selbstverständlich in an sich bekannter Weise auch eine rechnerische Verarbeitung der Meßwerte gemäß Fig. 3 möglich, welche unmittelbar zu den Kurven gemäß Fig. 4 und 5 führt. Darüberhinaus enthält Fig. 3 aber noch weitere Detailinformationen entsprechend dem genauen Verlauf der Amplitude bei jeder einzelnen Abtastung, woraus sich gegebenenfalls weitere Erkenntnisse über Einzelheiten des Ablaufs der Konsistenzänderungen in der Fluidprobe 3a entnehmen und Rückschlüsse auf die diesen Änderungen zugrundeliegenden Faktoren ziehen lassen.

Mit einer Anordnung gemäß Fig. 2 können die Meßkurven gemäß Fig. 4 und 5 auch unmittelbar erhalten werden. Hierzu ist ein Steuerteil 13 vorgesehen, welches eine automatische Nachregelung der Anregungsfrequenz des Schwingantriebes 8 auf die momentane Resonanzfrequenz des Schwingsystems ergibt. Als Ist-Größe wird hierzu die am Schwingungssensor 10 ermittelte Amplitude zum Steuerteil 13 geführt, welches die Anregungsfrequenz dauernd so nachstellt, daß der momentane Wert der Amplitude ein Maximum wird. Dies bedeutet, daß am Schwingungssensor 10 stets - im Bedarfsfall auch intermittierend - die Resonanzamplitude abgegriffen wird, gewissermaßen also stets die maximale Amplitude der in Fig. 3 veranschaulichten Kurven vorliegt. Damit ergibt sich gemäß Fig. 5 der Verlauf der maximalen Resonanzamplitude über der Zeit, deren Abfall der Dämpfung entspricht. Durch Bildung des Umkehrwertes der Maximalamplitude gemäß Fig. 5 kann selbstverständlich auch direkt die Dämpfung, die entsprechend ansteigt, aufgezeichnet werden, die ein unmittelbares Maß für die Viskosität des Probefluids 3a ist. Gleichzeitig kann an Leitungen 12a über der Zeit – im Beispielsfalle über derselben Zeitabszisse des gemeinsamen X-Y-Schreibers 14 – festgehalten werden, welche Anregungsfrequenz das Steuerteil 13 jeweils einstellt, was, da die Anregungsfrequenz ja jeweils auf die Resonanzfrequenz des Schwingsystems eingeregelt wird, dem Verlauf der Resonanzfrequenz des Schwingsystems gemäß Fig. 4 entspricht. Auf diese Weise ergeben sich die Kurven gemäß Fig. 4 und 5 unmittelbar aus der Meßanordnung und können beispielsweise auf zwei verschiedenen oder einem gemeinsamen Schreiber festgehalten werden, so daß auf diese Weise ebenfalls Beginn und Verlauf der Änderungen der Elastizität getrennt von Änderungen der Viskosität ermittelt werden. In den Fig. 4 und 5 sind im Auslaufbereich der Meßkurven gestrichelte Bereiche 15 und 16 veranschaulicht, innerhalb deren der Kurvenverlauf bei einer Schwingung in Normalblut liegt. In dem zeitlich davorliegenden Bereich können aus den Meßkurven möglichst genaue Werte für den Zeitpunkt $T_1$ des Beginns der Veränderung des Elastizitätsmoduls des Probefluids 3a und für den Zeitpunkt $T_2$ des Beginns der Änderung der Viskosität des Probefluids 3a sowie für den Anstieg $\alpha$ des Elastizitätsmoduls und den Anstieg $\beta$ der Dämpfung (entsprechend dem Abfall der Amplitude) ermittelt werden.

Mit dem erfindungsgemäßen Verfahren kann somit in Ab-

0015550

hängigkeit von den jeweiligen Erfordernissen der Messung im Einzelfall eine Kurvenschar gemäß Fig. 3 oder eine Kurvenausbildung gemäß den Fig. 4 und 5 erhalten werden, so daß alle relevanten Änderungen des visko-elastischen Verhaltens des Probefluids 3a schnell und sicher getrennt voneinander ermittelt werden können.

Dr. Eduard Fresenius

Chemisch-pharmazeutische

Industrie KG

6380 Bad Homburg

PATENTANWÄLTE

R.-A. KUHNEN', DIPL ING.
W. LUDERSCHMIDT'', DR. ING. CHEM.
P.-A. WACKER', DIPL ING. DIPL WIRTSCH ING.

55 FR 0330 2/st

## Patentansprüche

1. Verfahren zur Bestimmung der visko-elastischen Eigenschaften von Fluiden und/oder polymerisierenden Gemischen, insbesondere von gerinnenden Flüssigkeiten wie Blut oder Blutplasma, und deren zeitlichen Änderungen, bei dem ein Körper in eine Fluidprobe taucht und der Körper oder ein die Fluidprobe aufnehmendes Probengefäß elastisch abgestützt und zumindest in der Nähe der Eigenfrequenz des durch den Körper oder das Probengefäß mit zugehöriger Abstützung gebildeten Schwingsystems zur Schwingung angeregt wird, dadurch gekennzeichnet, daß die Resonanzfrequenz und/oder die Amplitude in der Resonanzfrequenz jeweils erfaßt werden.

2. Verfahren nach Anspruch 1, dadurch gekennzeichnet, daß die Erfassung der Amplitude ausschließlich in der Resonanzfrequenz des Schwingsystems durchgeführt wird.

BÜRO 6370 OBERURSEL
LINDENSTRASSE 10
TEL 06171 56849
TELEX 4186343 real d

BÜRO 8050 FREISING
SCHNEGGSTRASSE 3-5
TEL 08161 62091
TELEX 526547 pawa d

ZWEIGBÜRO 8390 PASSAU
LUDWIGSTRASSE 2
TEL 0851 3ee1e

——— TELEGRAMMADRESSE PAWAMUC — POSTSCHECK MÜNCHEN 1361-856 ———

3. Verfahren nach Anspruch 1 oder 2, dadurch gekennzeichnet, daß die Anregungsfrequenz zumindest quasikontinuierlich der Resonanzfrequenz des Schwingsystems nachgestellt wird.

4. Verfahren nach Anspruch 3, dadurch gekennzeichnet, daß die erforderliche Nachstelländerung der Anregungsfrequenz oder einer davon abgeleiteten Meßgröße zusätzlich zur jeweiligen Amplitude als Funktion der Zeit aufgezeichnet wird.

5. Verfahren nach Anspruch 1 oder 2, dadurch gekennzeichnet, daß die Anregungsfrequenz in gegenüber der Geschwindigkeit der zu messenden zeitlichen Änderung der visko-elastischen Eigenschaften kurzen Zeitabständen den Resonanzfrequenzbereich des Schwingsystems zur Abtastung überfährt.

6. Verfahren nach Anspruch 5, dadurch gekennzeichnet, daß die Abtastung mit vorbestimmter Änderung der Anregungsfrequenz durchgeführt wird.

7. Verfahren nach Anspruch 5 oder 6, dadurch gekennzeichnet, daß die Abtastfrequenz im Falle von Blut oder Blutplasma als Fluid zwischen etwa 2 und 0,005 Hz gewählt wird.

8. Verfahren nach einem der Ansprüche 5 bis 7, dadurch gekennzeichnet, daß der Frequenzbereich der Abtastung zwischen dem 0,1-fachen und dem 3-fachen, bevorzugt zwischen etwa dem 0,8-fachen und dem 1,8-fachen der Anfangsresonanzfrequenz des Schwingsystems gewählt wird.

9. Verfahren nach einem der Ansprüche 6 bis 8, dadurch gekennzeichnet, daß die Abtastkurven über der Zeit beginnend zu Anfang jedes Abtastlaufes einander überlagernd aufgezeichnet werden.

10. Verfahren nach einem der Ansprüche 1 bis 9, dadurch gekennzeichnet, daß die Anfangsresonanzfrequenz des Schwingsystems zwischen etwa 10 und 1000 Hz, bevorzugt zwischen etwa 25 und 150 Hz, insbesondere bei etwa 80 Hz gewählt wird.

11. Vorrichtung zur Bestimmung der visko-elastischen Eigenschaften von Fluiden und/oder polymerisierenden Gemischen, insbesondere von gerinnenden Flüssigkeiten wie Blut oder Blutplasma, und deren zeitlichen Änderungen, bei dem eine Fluidprobe in einen Zwischenraum zwischen einem Probengefäß und einem in das Probengefäß eintauchenden Körper einfüllbar und Probengefäß oder Körper zur Bildung eines Schwingsystems elastisch gelagert und mittels eines Schwingantriebs schwingend antreibbar ist, sowie die momentane Amplitude des elastisch gelagerten Teiles laufend durch einen Schwingungssensor meßbar ist, insbesondere zur Durchführung des Verfahrens nach wenigstens einem der Ansprüche 1 bis 10, dadurch gekennzeichnet, daß dem Schwingantrieb (8) ein Steuerteil (13) zur Frequenzeinstellung zugeordnet ist, dessen die Anregungsfrequenz des Schwingsystems bildende Steuerfrequenz periodisch einen die Resonanzfrequenz des Schwingsystems überdeckenden Bereich überfährt.

12. Vorrichtung nach Anspruch 11, dadurch gekennzeichnet, daß das Steuerteil (13) eine Kippstufe aufweist.

13. Vorrichtung zur Bestimmung der visko-elastischen Eigenschaften von Fluiden und/oder polymerisierenden Gemischen, insbesondere von gerinnenden Flüssigkeiten wie Blut oder Blutplasma, und deren zeitlichen Änderungen, bei dem eine Fluidprobe in einen Zwischenraum zwischen einem Probengefäß und einem in das Probengefäß eintauchenden Körper einfüllbar und Probengefäß oder Körper zur Bildung eines Schwingsystems elastisch gelagert und mittels eines Schwingantriebs schwingend antreibbar ist, sowie die momentane Amplitude des elastisch gelagerten Teiles
laufend durch einen Schwingungssensor meßbar ist, insbesondere zur Durchführung des Verfahrens nach wenigstens einem der Ansprüche 1 bis 10, dadurch gekennzeichnet, daß dem Schwingantrieb (8) ein Steuerteil
(13) zur Frequenzeinstellung zugeordnet ist, dessen
die Anregungsfrequenz des Schwingsystems bildende
Steuerfrequenz durch Zuführung der Meßsignale des
Schwingungssensors (10) auf die momentane Resonanzfrequenz des Schwingsystems einstellbar ist.

14. Vorrichtung nach Anspruch 13, dadurch gekennzeichnet, daß die Steuerfrequenz des Steuerteiles (13)
über eine Regelschaltung so einstellbar ist, daß
das Meßsignal des Schwingungssensors (10) ein Maximum ist.

15. Vorrichtung nach wenigstens einem der Ansprüche 11
bis 14, dadurch gekennzeichnet, daß der Schwingantrieb (8) durch Elektromagnete (8a) gebildet ist,
und daß zwischen das Steuerteil (13) und wenigstens
einen der Elektromagnete (8a) ein Phasenschieber
(11b) geschaltet ist.

16. Vorrichtung nach wenigstens einem der Ansprüche 11
bis 15, dadurch gekennzeichnet, daß der Körper (2)
am unteren Ende eines elastischen Stabes (5) vorzugsweise leicht auswechselbar gehalten ist.

17. Vorrichtung nach wenigstens einem der Ansprüche 11
bis 16, dadurch gekennzeichnet, daß das Probengefäß (1) vorzugsweise leicht auswechselbar am Gerätegestell (4) befestigt ist.

18. Vorrichtung nach wenigstens einem der Ansprüche 11
bis 17, dadurch gekennzeichnet, daß ein Mehrfachhalter für eine Mehrzahl von Probengefäßen (1) für eine Meßreihe vorgesehen ist.

19. Vorrichtung nach wenigstens einem der Ansprüche 11 bis 18, dadurch gekennzeichnet, daß der Schwingungssensor (10) ein Hallgenerator ist.

20. Vorrichtung nach einem der Ansprüche 16 bis 19, dadurch gekennzeichnet, daß der Schwingungssensor (10) in der Nachbarschaft des den Körper (2) tragenden Endes des Stabes (5) vorgesehen ist.

21. Vorrichtung nach einem der Ansprüche 15 bis 20, dadurch gekennzeichnet, daß der Schwingungssensor (10) mittels einer Magnetfeldabschirmung (9) gegen die Einwirkung benachbart angeordneter Elektromagnete (8a) geschützt ist.

0015550

Fig.1

Fig. 2

entanwälte
:N & WACKER
ıstr. 3, Postfach

0015550

Fig. 4

α

T₁

15

Fig. 5

A max

β

T₂

16

Fig. 6

γ₂

γ₁

T

tentanwälte
EN & WACKER
gstr. 3, Postfach

Fig. 3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | JOURNAL OF APPLIED PHYSICS, Band 49, Nr. 3, März 1978, New York, US, W.H. ROBINSON et al.: "Piezoelectric method of determining viscosity at 40 KHz", Seiten 1070-1076 <br><br> * Seite 1070, linke Spalte, Zeilen 26-37 * <br><br> -- | 1 |
| | GB - A - 851 621 (FARBENFABRIKEN BAYER AG) <br><br> * Seite 1, Zeilen 14-28 * <br><br> -- | 1,2 |
| | GB - A - 1 233 881 (N.V. TOOLS LTD.) <br><br> * Seite 1, Zeilen 14-32 * <br><br> -- | 1 |
| | US - A - 3 943 753 (F.N. SIMON) <br> * Spalte 1, Zeile 62 bis Spalte 2, Zeile 16; Spalte 5, Zeilen 13-28; Figur 1 * <br><br> -- | 1,5, 11 |
| | US - A - 3 349 604 (W.B. BANKS) <br> * Spalte 2, Zeile 56 bis Spalte 5, Zeile 1; Figur 2 * <br> *. <br><br> -- | 1 |
| A | FR - A - 2 370 971 (PROBIO DMS) <br> * Seite 12, Zeilen 1-25; Figur 2 * <br><br> -- <br><br> ./. | 1 |

**KLASSIFIKATION DER ANMELDUNG (Int Cl )**

G 01 N 11/16
33/48

**RECHERCHIERTE SACHGEBIETE (Int Cl )**

G 01 N 11/16
11/10
33/48

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 09-06-1980 | ANTHONY |

| Kategorie | Kennzeichnung des Dokuments mit Angabe, soweit erforderlich, der maßgeblichen Teile | betrifft Anspruch |
|---|---|---|
| A | US - A - 4 026 671 (S.L. SIMONS et al.) <br><br> * Spalte 2, Zeile 31 bis Spalte 3, Zeile 45; Figur 2 * <br><br> -- | 1 |
| A | US - A - 3 225 588 (J.F. MOULIN et al.) <br><br> * Spalte 1, Zeilen 10-23; Figur 1 * <br><br> -- | 1 |
| P | US - A - 4 154 093 (N.D.P. SMITH et al.) <br><br> * Spalte 1, Zeile 57 bis Spalte 2, Zeile 55 * <br><br> -- | 1 |
| P | GB - A - 2 004 376 (E. FRESENIUS CHEM-PHARM INDUSTRIE KG) <br><br> * Seite 1, Zeilen 54-96 * <br><br> & FR - A - 2 402 874 <br> DE - A - 2 741 060 <br><br> ---- | 1 |

**EINSCHLÄGIGE DOKUMENTE**

KLASSIFIKATION DER ANMELDUNG (Int.Cl. 3)

RECHERCHIERTE SACHGEBIETE (Int. Cl. 3)